# EUROPEAN PATENT APPLICATION

(11) **EP 3 881 890 A1**
(43) Date of publication of application: **22.09.2021**
(21) Application number: 20163794.9
(22) Date of filing: 18.03.2020
(51) Int. Cl.: A61N 1/36, A61N 1/372, G16H 40/00

(54) **A PLANNING AND/OR CONTROL SYSTEM FOR A NEUROMODULATION SYSTEM**

(71) Applicant: ONWARD Medical B.V., 5656 AE Eindhoven (NL)
(72) Inventor: BROUNS, Robin, Eindhoven (NL); CABAN, Miroslav, 1020 Renens (CH)
(74) Representative: DTS Patent- und Rechtsanwälte Schnekenbühl und Partner mbB

(57) **Abstract**

A planning and/or control system (10, 110, 210) for a neuromodulation system, especially a neurostimulation system for a patient, comprising
- a stimulation pattern programming module (12, 112, 212) configured and arranged for receiving and processing data on stimulation parameters and a time sequence of stimulation parameters,
- a graphical presentation module (14, 114, 214) configured and arranged for providing graphical information about the stimulation parameters and the time sequence of the stimulation parameters.

## Description

The present invention relates to control systems for tissue stimulating systems, in particular a planning and/or control system for a neuromodulation system, especially a neurostimulation system for a patient.

The spinal cord is an integral part of the central nervous system (CNS). Spinal cord injury (SCI), but also other disorders (e.g. stroke, multiple sclerosis, autonomic failure, autonomic neuropathy or cancer of the neurological tissue which impair operation of descending sympathetic pathways that normally facilitate control of autonomic functions) result in motor deficits. For instance, SCI interrupts the communication between the spinal cord and supraspinal centres, depriving these sensorimotor circuits from the excitatory and modulatory drives necessary to produce movement. However, SCI results also in sensory deficits and in autonomic dysfunctions. In particular, SCI results in disconnection of some, most, or all descending sympathetic pathways that carry signals responsible for regulating arterial blood pressure, heart rate and/or gut and/or bladder function.

Spinal cord stimulation (SCS) is a well-established neuromodulatory therapy not only for restoring locomotion/motoric function after spinal cord injury or central nervous diseases, but also for treating inter alia pain and/or restoring autonomic function.

A neuromodulation system, especially a neurostimulation system for a patient suffering from motoric dysfunction and/or autonomic dysfunction requires programming to define which stimulation settings need to be used to evoke certain muscles or muscle groups. Such muscles and/or muscle groups may be responsible for locomotion of the arms or legs, and/or responsible for e.g. bowel movement, sphincter control, bladder control and/or sexual function. Such programming of stimulation parameters may be performed by a clinical professional, a physiotherapist and/or the patient himself, and facilitated by a computer-driven application.

Once stimulation parameters have been found to stimulate certain muscles (or groups of muscles), the muscular activations need to be sequenced in time. For example, walking is defined by alternating stimulations of specific muscles on the right and on the left leg. Similarly, any kind of other locomotion, e.g. running, swimming, cycling, rowing, can be thought of as a timeline or sequence of muscular stimulations.

US2014172045A1 discloses a method for programming a neurostimulator. One or more control elements may be actuated to select the series of steps from a plurality of series of steps stored in a memory of an external control device. One or more control elements may be actuated during the performance of the series of steps in order to cause one of the steps to pause, stop, restart, skip, or repeat. The series of steps may be a series of pre-programming steps, and the method may further include programming the neurostimulator after the series of pre-programming steps is performed. An external device for programming the neurostimulator includes control circuitry configured for automatically performing the series of steps, and a user interface including the one or more control elements configured for being actuated. The control device also includes the memory for storing the plurality of series of steps.

EP3328481A1 disclose a neurostimulation system including a programming control circuit and a user interface. The programming control circuit may be configured to generate a plurality of stimulation parameters controlling delivery of neurostimulation pulses according to one or more neurostimulation programs each specifying a pattern of the neurostimulation pulses. The user interface includes a display screen, a user input device, and a neurostimulation program circuit. The neurostimulation program circuit may be configured to allow for construction of one or more pulse trains (PTs) and one or more train groupings (TGs) of the one or more neurostimulation programs, and to allow for scheduling of delivery of the one or more neurostimulation programs, using the display screen and the user input device. Each PT includes one or more pulse blocks each including a plurality of pulses of the neurostimulation pulses. Each TG includes one or more PTs.

It is an object of the present invention to provide a planning and/or control system for a neuromodulation system, especially a neurostimulation system for a patient that enables optimal sequence of responses to stimulation, e.g. on smooth movements of a patient, by facilitating time sequencing of stimulation parameters.

This object is solved according to the present invention by a planning and/or control system for a neuromodulation system with the features of claim 1. Accordingly, a planning and/or control system for a neuromodulation system, especially a neurostimulation system for a patient, comprising
- a stimulation pattern programming module configured and arranged for receiving and processing data on stimulation parameters and a time sequence of stimulation parameters,
- a graphical presentation module configured and arranged for providing graphical information about the stimulation parameters and the time sequence of the stimulation parameters.

The invention is based on the basic idea that in the context of neuromodulation, especially neurostimulation, stimulation parameters need to be adapted to specific needs of a patient. In particular, the stimulation parameters may be patient specific and/or require frequent modifications and/or precise timing of start and stop. In particular some patients may move faster/slower (such as walking) than others, requiring neurostimulation to succeed in a respective faster or slower fashion. Furthermore, the support required from stimulation may vary on the patient's specific pathological symptoms. Thus, the stimulation sequence may involve different muscles or muscle groups and they may be required at different moments or for different durations during the exercise or stimulation program. On some days, a patient may require longer (or shorter) stimulation of a certain muscle than on other days. Also, patients may be expected to progress and show improvements, placing ever new requirements on their stimulation patterns. The use of a general concept including a stimulation pattern programming module and a graphical presentation module combined into one strategy and made available for a neuromodulation system, especially a neurostimulation system for a patient enables fast, simplified and efficient planning of a precise neuromodulation of a patient, in particular in that the outcome of the stimulation is close to the respective physiological function of a healthy and/or uninjured individual and/or patient specific.

A stimulation block may also be referred to as muscle group.

In particular, the system further may comprise at least one of a display, a controller, a programmer, a communication module, a telemetry module, a stimulation device, an electrode, a sensor and/or a sensor network.

The stimulation pattern programming module may be or may comprise an interface module configured and arranged for receiving user-based stimulation parameters and/or a user-based time sequence of the stimulation parameters. In particular, the interface module may enable that a user, e.g. a therapist, a physiotherapist, a physician, a trainer, a medical professional and/or a patient directly provides modification of the stimulation parameters to enable patient specific modification, e.g. by manually sequencing of stimulation patterns. In particular, the interface module may be or may comprise a graphical user interface and/or a mouse and/or a trackball and/or a joystick. In particular, the graphical user interface may comprise a display and/or a touch screen and/or a touch pad. In particular, the interface module may be configured and arranged for allowing a user to actuate at least one control element. Such a system also has the advantage that it is easy for the user to use, so that no time-consuming training is necessary.

In particular, the user may manually place blocks on a timeline for defining stimulation parameters, move these, increase or decrease their timing. This may be done on a computer system. Furthermore, templates may be provided for certain exercises or stimulation programs (e.g. for restoring autonomic function) and for patients with specific residual motor-functions and/or autonomic functions.

Furthermore, the user may manually change stimulation parameters and/or the time sequence of stimulation parameters and/or the blocks on the timeline by acting on control elements configured for being actuated by the user, including but not limited to axes, points, knots, buttons, arrows, hand signals, emojis, crosses and/or windows and/or text and/or shortcuts.

In particular, the user may manually change stimulation parameters and/or the time sequence of stimulation parameters and/or the blocks on the timeline by acting on an avatar configured and arranged for being actuated by the user.

The system may be configured such that the stimulation parameters and/or the time sequence of the stimulation parameters is modulated dependent on modulation of the graphical information about the stimulation parameters and the time sequence of the stimulation parameters. In particular, this may enable rapid modification of stimulation parameters and subsequent stimulation of a patient, enabling rapid therapy progress, with a therapy specifically adapted to the patient's needs.

In particular, the stimulation parameters and/or the time sequence of the stimulation parameters may be modulated in real-time and/or close to real-time. In other words, the stimulation parameters and/or the time sequence of the stimulation parameters may be modulated during stimulation and/or task execution.

Alternatively, and/or additionally, the stimulation parameters and/or the time sequence of the stimulation parameters may be modulated before stimulation is provided (pre-programming of stimulation parameters and/or the time sequence of the stimulation parameters).

Further, the stimulation pattern programming module may be or may comprise a computer-assisted module configured and arranged for updating stimulation parameters and/or the time sequence of stimulation parameters based on feedback information provided by at least one external module and/or a set of instructions. Such feedback information and/or instructions may fine-tune the start and/or stop and/or ramping up and/or ramping down and/or repetitions, etc. of stimulation patterns or control the position of a muscle and/or stimulation block.

In particular, the computer-assisted module may be connected to at least one external module. The external module may be or may comprise at least one sensor and/or sensor network providing feedback, e.g. of the patient, a patient's position and/or his environment and/or his physiology/pathophysiology.

Alternatively, and/or additionally, the computer assisted module may be configured and arranged for providing at least one set of instructions, e.g. at least one algorithm, automatically altering and/or updating stimulation parameters and/or time sequencing of stimulation parameters. In particular, this may have the advantage that the stimulation parameters and/or time sequence of stimulation parameters are optimized during an ongoing motion and/or behavior of a patient, by data derived from the patient himself and/or his environment and/or a set of instructions, enabling efficient and fast rehabilitation.

The information provided by external modules and/or the at least one set of instructions may be used by the system, e.g. by the computer-assisted module and/or another processing module, to alter the stimulation parameters and/or time sequencing of stimulation parameters, possibly in addition to manually setting stimulation parameters and/or time sequencing of stimulation parameters by a user.

Further, external modules may be used to provide (feedback) information that may be communicated to the user, e.g. acoustically and/or visually, based on which the user may program the stimulation parameters and/or time sequencing of stimulation parameters, with or without automatically updates. For instance, a live EMG trace may be displayed in the background of stimulation parameters and/or time sequencing of stimulation parameters, or the video of the patient may be displayed behind an avatar, or auditory tones / spoken messages may be provided that inform the user (e.g. therapist and/or patient) of stimulation events. Such information may allow a user to update the stimulation parameters and/or time sequencing of stimulation parameters. In other words, the feedback information may aid the user to perform the programming.

In particular, the computer assisted module may update the stimulation parameters and/or the time sequence of stimulation parameters in real-time or close to real-time, i.e. with minimum delay.

A sensor and/or sensor network may be or may comprise at least one of an inertial measurement unit (IMU), an optical sensor, a camera, a piezo element, a velocity sensor, an accelerometer, a magnetic sensor, a torque sensor, a pressure sensor, a displacement sensor, a contact sensor, an EMG sensor, a goniometer, a hall sensor and/or a gyroscope and/or motion tracking video camera, or infra-red camera.

In particular, the system may be a closed-loop system or an open-loop system.

It is also possible that the system allows both closed-loop or open-loop functionality. In this regard, the user may switch between these options or there may be routines or control elements that can do or propose such a switch from closed-loop to open-loop and vice versa.

In particular, the system may be used online and/or offline.

In particular, the graphical presentation module may be configured and arranged for providing graphical information about the time sequence of the stimulation parameters as a timeline, a graph, a plot, a table, a circular pattern and/or a clock. Thus, the time sequence of the stimulation parameters is presented to the user in a graphically appealing and easy layout, which can be easily understood by the user. This has the advantage that, due to the simplicity of the operation and the layout, the number of errors that occur when operating is reduced.

To provide the user a better guidance for improving the patient's stimulation parameters, e.g. camera recordings (provided by a camera comprised by the external module) and/or frames could be superimposed onto optimal outcome patterns.

Optimal outcome patterns could be movements of a healthy subject.

As such, the user could see which muscles need more and/or less activation to match e.g. an optimal walking pattern.

This could be applied to kind of locomotion activity and/or motoric function.

In particular, physiological measurements by sensors may be added and/or superimposed to stimulation parameters.

In particular, sensors may be applied on a patient to measure when muscles are activated voluntarily by the patient, and this could be displayed in the same time sequence of stimulation parameters. In general, feedback information provided by sensors may be used to better define stimulation parameters and to align with the patient's voluntary activity.

In particular, timings of start and stop of stimulation of a certain muscle needs to be accurate. For instance, during locomotion, muscular action succeeds in rapid fashion and muscles may need to be timed to each other sometimes down to e.g. 10ms to 80ms. Providing graphical information about the time sequence of stimulation parameters, e.g. as a timeline allows the user to judge the patient's movements visually. Further, as for e.g. locomotion, many muscles and/or stimulation blocks and/or stimulation parameters are involved, it may be challenging for a user to bring everything together and produce a therapeutic, effective timeline of stimulation parameters. In particular, providing graphical information of the time sequence of stimulation parameters, as a timeline, a graph, a plot, a table, a circular pattern, a clock may enable to bring everything together and produce a therapeutic effective timeline. Overall, rapid and correct modification of stimulation parameters and subsequent stimulation of a patient, enabling rapid therapy progress, with a therapy specifically adapted to the patient's needs is enabled.

Additionally, and/or alternatively, a combination of different layouts may be possible, provided as graphical information in black and white and/or color.

The stimulation parameters may comprise amplitude, frequency, and/or pulse width of the stimulation of at least one muscle and/or at least one stimulation block and the time sequence of stimulation parameters may comprise start, stop, up ramping, down ramping, duration, repetition and/or cycles of stimulation of at least one muscle and/or stimulation block. This may enable that all stimulation parameters required for a complex process, such as walking, standing up, sitting down, cycling, swimming and/or grasping may be exactly defined, finally enabling smooth movements and/or defined physiological functions.

In particular, the stimulation parameters may comprise up-ramping and/or down-ramping. Ramping up and/or ramping down may be important to facilitate gradual transitioning of stimulation, rather than an immediate violent application of stimulation that the patient overcome with.

A timeline of stimulation parameters may be repeated and/or cycling to allow for a constant locomotion to be performed, e.g. walking, running, cycling, swimming and/or rowing. In constant locomotion, the timeline of stimulation parameters should behave like an automatically replay in these exercises. Similarly, stimulation programs for autonomic functions may be created using such repeated or cyclic timelines.

In particular, it may be possible that the timeline of stimulation parameters
- Runs once and then stimulation is stopped;
- Runs once but keeps stimulating the last stimulation block of the timeline indefinitely until manually stopped by a user or by a sensor that detects patient-voluntary initiation of another type of locomotion. This may be beneficial for e.g. standing up, where at the end it may be required to facilitate stimulation to keep the patient standing upright;
- Runs indefinitely until manually stopped by a user.

The system may further comprise an acoustic module being configured and arranged for providing acoustic information about the stimulation parameters and/or the time sequence of the stimulation parameters. This has the advantage that a user may focus visually on the patient's movements and simply rely on auditory cues to understand a stimulation provided by a neuromodulation system. Focusing on the patient may allow the user to more quickly intervene to guarantee the patient's safety. Beyond, this may allow the patient to modulate his or her self-invoked movements to align with stimulations provided, to the benefit of therapy. In other words, this may aid the user to perform the programming.

In particular, the system may play an auditory cue/tone as soon as stimulation of a stimulation block and/or a muscle is initiated and/or stopped. In particular, the system that produces the auditory cue may not be limited to the system providing the means for creation of the stimulation pattern.

The auditory cue may be a simple tone and/or a single frequency and/or multiple frequencies or may even be a vocal cue such as a voice reading out the stimulation parameters. The vocals may also provide a count-down before stimulation occurs. This may help the patient to align and prepare his self-invoked movements to align with these stimulations. Overall, efficient therapy is enabled.

According to the present invention a method is disclosed, the method characterized in that the method is performed with the system of any of claims 1-8.

In particular, the method may be a method for performing neuromodulation, the method comprising at least the following steps:
- receiving and processing stimulation parameters and a time sequence of the stimulation parameters,
- providing graphical information about the stimulation parameters and the time sequence of the stimulation parameters.

The method may further comprise the step of receiving user-based stimulation parameters and/or a user-based time sequence of the stimulation parameters.

The method may further comprise the step of modulating the stimulation parameters and/or the time sequence of the stimulation parameters by modulation of the graphical information about the stimulation parameters and the time sequence of the stimulation parameters.

The method may further comprise the step of updating stimulation parameters and/or the time sequence of stimulation parameters based on feedback information and/or a set of instructions.

In particular, the graphical information about the time sequence of the stimulation parameters is provided as a timeline, a graph, a plot, a table, a circular pattern and/or a clock.

The method may further comprise the step of providing acoustic information about the stimulation parameters and/or the time sequence of the stimulation parameters.

Further details and advantages of the present invention shall now be disclosed in connection with the drawings.

It is shown in
- Fig. 1: a schematical overview of an embodiment of the planning and/or control system according to the present invention, with which the method according to the present invention can be performed;
- Fig. 2: a schematical overview of a further embodiment of the planning and/or control system according to the present invention, with which the method according to the present invention can be performed;
- Fig. 3: a schematical overview of a further embodiment of the planning and/or control system according to the present invention, with which the method according to the present invention can be performed;
- Fig. 4: an example of graphical information provided by the graphical presentation module according to the present invention;
- Fig. 5: a further example of graphical information provided by the graphical presentation module according to the present invention;
- Fig. 6: a further example of graphical information provided by the graphical presentation module according to the present invention;
- Fig. 7: a further example of graphical information provided by the graphical presentation module according to the present invention;
- Fig. 8,: an example of how a user can interact on a touchscreen with a block B on the timeline, according to the present invention;
- Fig. 9: an example of a timeline embodied as a circle that repeats itself, according to the present invention;
- Fig. 10: an example of how acoustic information is provided, according to the present invention;
- Fig. 11: an example of a schematical embodiment of how stimulation parameters could be updated according to the present invention;
- Fig. 12: an example of superimposing camera recordings on intended locomotion of a patient, according to the present invention;
- Fig. 13: a further example of adding (and/or superimposing) physiological measurements to support block B positioning, according to the present invention; and
- Fig. 14: direct manipulation and/or modulation of a movement of a patient on an avatar, according to the present invention.

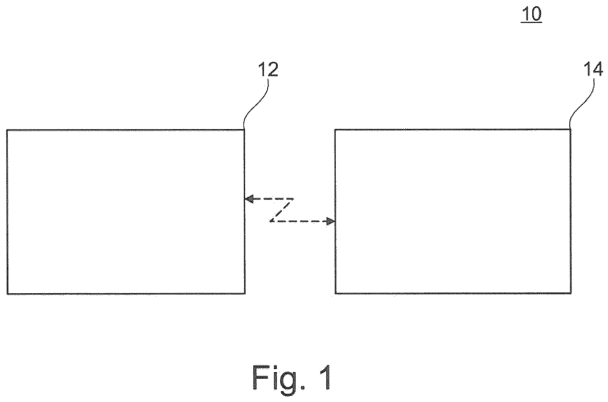

**Fig. 1** shows a schematical overview of an embodiment of the planning and/or control system 10 according to the present invention, with which the method according to the present invention can be performed.

In this embodiment, a planning and/or control system 10 for a neuromodulation system, especially a neurostimulation system for a patient is shown.

The system 10 comprises a stimulation pattern programming module 12.

The stimulation pattern programming module 12 is configured and arranged for receiving and processing data on stimulation parameters and a time sequence of stimulation parameters.

The system 10 further comprises a graphical presentation module 14.

The graphical presentation module 14 is configured and arranged for providing graphical information about the stimulation parameters and the time sequence of the stimulation parameters.

In this embodiment, the stimulation pattern programming module 12 and the graphical presentation module 14 are connected.

In this embodiment, the stimulation pattern programming module 12 and the graphical presentation module 14 are connected by a wireless link.

In this embodiment, the connection between the stimulation pattern programming module 12 and the graphical presentation module 14 is a bidirectional connection.

However, also unidirectional and/or cable-bound connections are generally possible.

In this embodiment, the stimulation pattern programming module 12 receives and processes data on stimulation parameters and a time sequence of stimulation parameters.

In this embodiment, the graphical presentation module 14 provides graphical information about the stimulation parameters and the time sequence of the stimulation parameters.

In other words, in this embodiment, the graphical presentation module 14 provides graphical information about the stimulation parameters and the time sequence of the stimulation parameters received and processed by the stimulation pattern programming module 12.

Not shown in Fig. 1 is that the stimulation parameters comprise amplitude, frequency, and/or pulse width of the stimulation of at least one muscle and/or at least one stimulation block and the time sequence of stimulation parameters comprises start, stop, up ramping, down ramping, duration, repetition and/or cycles of stimulation of at least one muscle and/or stimulation block.

Not shown in Fig. 1 is that the graphical presentation module 14 provides graphical information about the time sequence of the stimulation parameters as a timeline T, a graph, a plot, a table, a circular pattern and/or a clock.

Also not shown in Fig. 1 is that the neuromodulation system, especially the neurostimulation system for a patient could comprise at least one of a display, a controller, a programmer, a communication module, a telemetry module, a stimulation device, an electrode, a sensor and/or a sensor network.

In general, the system 10 could perform a method for planning and/or controlling neuromodulation, especially neurostimulation for a patient, comprising at least the following steps:
- receiving and processing stimulation parameters and a time sequence of the stimulation parameters,
- providing graphical information about the stimulation parameters and the time sequence of the stimulation parameters.

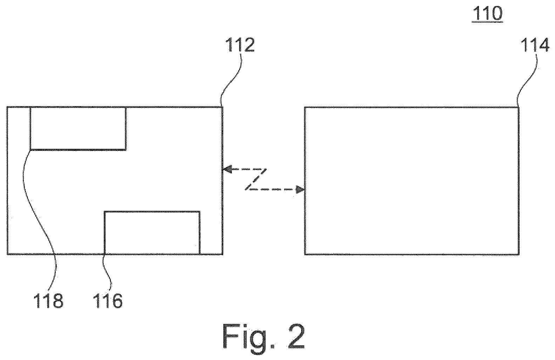

**Fig. 2** shows a schematical overview of a further embodiment of the planning and/or control system 110 according to the present invention, with which the method according to the present invention can be performed.

The system 110 comprises the structural and functional features as disclosed for planning and/or control system 10 disclosed in Fig. 1. The corresponding reference numbers in Fig. 2 for identical or similar elements or features correspond to the corresponding reference numbers of Fig. 1 and to reflect this, then this reference number is taken and increased by the value 100.

In this embodiment, the stimulation pattern programming module 112 comprises an interface module 116.

Not shown in Fig. 2 is that alternatively the stimulation pattern programming module 112 could be an interface module 116.

In this embodiment, the stimulation pattern programming module 112 further comprises a computer-assisted module 118.

Not shown in Fig. 2 is that alternatively, the stimulation pattern programming module 112 may be a computer-assisted module 118.

The interface module 116 is configured and arranged for receiving user-based stimulation parameters and/or a user-based time sequence of the stimulation parameters.

The computer assisted module 118 is configured and arranged for updating stimulation parameters and/or the time sequence of stimulation parameters based on feedback information.

Not shown in Fig. 2 is that the interface module 116 is connected to the stimulation pattern programming module 112 by a wireless link, enabling bidirectional connection.

Not shown in Fig. 2 is that alternatively, the interface module 116 may be connected to the stimulation pattern programming module 112 by a cable-bound and/or unidirectional connection.

Not shown in Fig. 2 is that the computer-assisted module 118 is connected to the stimulation pattern programming module 112 by a wireless link, enabling bidirectional connection.

Not shown in Fig. 2 is that alternatively, the interface module 118 may be connected to the stimulation pattern programming module 112 by a cable-bound and/or unidirectional connection.

Not shown in Fig. 2 is that the interface module 116 and the computer-assisted module 118 are also connected.

in this embodiment, the stimulation parameters and/or the time sequence of the stimulation parameters are modulated dependent on modulation of the graphical information about the stimulation parameters and the time sequence of the stimulation parameters.

Not shown in Fig.2 is that the system 10, 110 could alternatively comprise either an interface module 116 or a computer-assisted module 118.

In this embodiment, the interface module 116 receives user-based stimulation parameters and/or a user-based time sequence of the stimulation parameters.

In this embodiment, the stimulation parameters and/or the time sequence of the stimulation parameters is modulated dependent on modulation of the graphical information about the stimulation parameters and the time sequence of the stimulation parameters.

In general, the system 110 could perform a method for planning and/or controlling neuromodulation, especially neurostimulation for a patient, comprising at least the following steps:
- receiving and processing stimulation parameters and a time sequence of the stimulation parameters,
- providing graphical information about the stimulation parameters and the time sequence of the stimulation parameters.

The method could further comprise the step of modulating the stimulation parameters and/or the time sequence of the stimulation parameters by modulation of the graphical information about the stimulation parameters and the time sequence of the stimulation parameters.

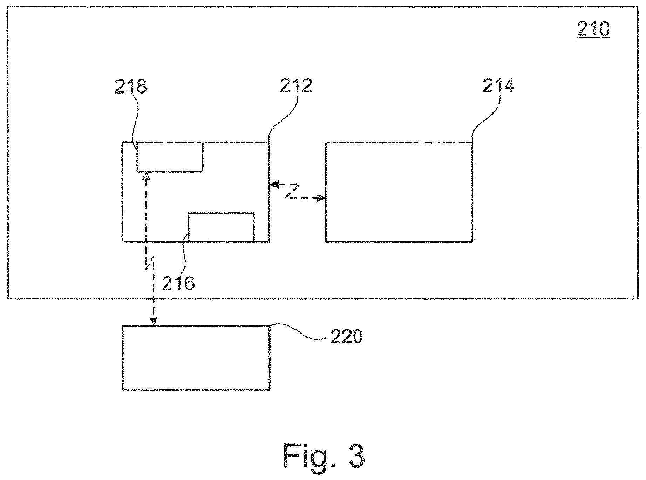

**Fig. 3** shows a schematical overview of a further embodiment of the planning and/or control system 210 according to the present invention, with which the method according to the present invention can be performed.

The system 210 comprises the structural and functional features as disclosed for the planning and/or control system 110 disclosed in Fig. 2. The corresponding reference numbers in Fig. 3 for identical or similar elements or features correspond to the corresponding reference numbers of Fig. 2 and to reflect this, then this reference number is taken and increased by the value 100.

In this embodiment, the system 210 is connected to an external module 220.

in this embodiment, the computer-assisted module 218 is connected to the external module 220.

In this embodiment, the computer-assisted module 218 is connected to the external module 220 via a bidirectional connection.

In this embodiment, the computer assisted module 218 and the external module 220 are connected by a wireless link.

However, in an alternative embodiment, a cable-bound and/or unidirectional connection could also be possible.

In an alternative embodiment, the system 210 could be connected to more than one external module 220.

Not shown in Fig. 3 is that the external module 220 comprises a sensor.

Not shown in Fig. 3 is that the sensor measures motion of a patient.

Not shown in Fig. 3 is that sensor provides feedback information of a patient equipped with the sensor.

Not shown in Fig. 3 is that the stimulation parameters and/or the time sequence of stimulation parameters are updated based on the feedback information provided by the sensor.

Not shown in Fig. 3 is that the stimulation parameters and/or the time sequence of stimulation parameters are updated in real-time.

Not shown in Fig. 3 is that alternatively, the stimulation parameters and/or the time sequence of stimulation parameters could be updated closed to real-time, with minimum delay.

Not shown in Fig 3 is that additionally and/or alternatively, the computer-assisted module 218 could update the stimulation parameters and/or the time sequence of stimulation parameters based on a set of instructions.

Not shown in Fig. 3 is that the set of instructions could generally comprise at least one algorithm.

Not shown in Fig. 3 is that sensor and/or sensor network could be or could comprise at least one of an inertial measurement unit (IMU), an optical sensor, a camera, a piezo element, a velocity sensor, an accelerometer, a magnetic sensor, a torque sensor, a pressure sensor, a displacement sensor, a contact sensor, an EMG sensor, a goniometer, a hall sensor and/or a gyroscope and/or motion tracking video camera, or infra-red camera.

Not shown in Fig. 3 is that the system further comprises an acoustic module 230.

The acoustic module 230 is connected to the stimulation parameter programming module 212 and the graphical presentation module 214 via a wireless link.

The connection between the acoustic module 230 and the stimulation parameter programming module 212 and the graphical presentation module 214 is a bidirectional connection.

However, in an alternative embodiment, a unidirectional and/or cable-bound connection could be generally possible.

The acoustic module 230 provides acoustic information about the stimulation parameters and/or the time sequence of the stimulation parameters.

In general, the system 210 could perform a method for planning and/or controlling neuromodulation, especially neurostimulation for a patient, comprising at least the following steps:
- receiving and processing stimulation parameters and a time sequence of the stimulation parameters,
- providing graphical information about the stimulation parameters and the time sequence of the stimulation parameters.

The method could further comprise the step of modulating the stimulation parameters and/or the time sequence of the stimulation parameters by modulation of the graphical information about the stimulation parameters and the time sequence of the stimulation parameters.

The method could further comprise the step of updating stimulation parameters and/or the time sequence of stimulation parameters based on feedback information and/or a set of instructions.

The method could further comprise the step of providing acoustic information about the stimulation parameters and/or the time sequence of the stimulation parameters.

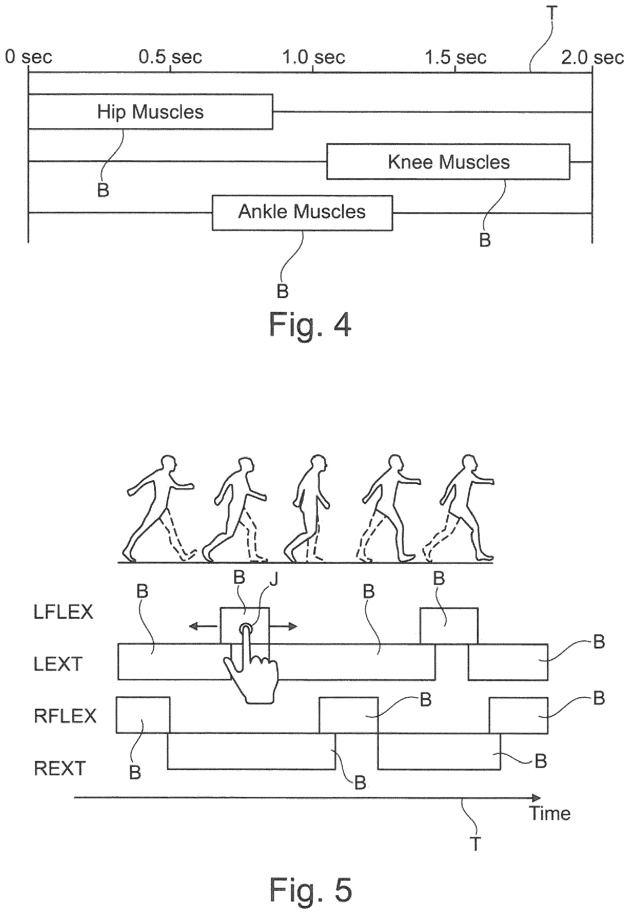

Fig. 4 shows an example of graphical information provided by the graphical presentation module 14, 114, 214 according to the present invention.

In particular, Fig. 4 shows a timeline T for defining a walking exercise for a patient.

In particular, Fig. 4 shows a generic abstract illustration of the timeline T concept with multiple rows.

The timeline T features stimulation parameters that represent certain muscle groups.

The timeline T features stimulation parameters that represent the muscle groups hip muscles, knee muscles and ankle muscles.

However, in general, every muscle and/or muscle group and/or stimulation block and/or muscle fiber could be shown.

Alternatively, the timeline T could feature stimulation parameters that represent certain muscle(s).

A muscle could be e.g. rectus femoris.

The timeline T features stimulation parameters that represent certain stimulation blocks.

In this embodiment, the stimulation parameters refer to a collection of muscles (1 or more) that are agonistic/synergistic to achieve a certain movement.

In this embodiment the movement is bending the hip.

In this embodiment, the stimulation parameters for hip muscles, knee muscles and ankle muscles are each set on the timeline T as a block B.

In this embodiment, the stimulation parameters for hip muscles are set on the timeline T as a block B to last from 0 seconds to 0.85 seconds, thereby lasting for 0.85 seconds.

Not shown in Fig. 4 is that a muscle-group could equally mean a synergistic muscle activation (e.g. withdrawal reflex) which is not necessarily focused on an isolated joint movement.

Not shown in Fig.4 is how blocks B could be created and/or altered, cf. Fig. 5.

**Fig. 5** shows a further example of graphical information provided by the graphical presentation module 14, 114, 214, according to the present invention.

In this embodiment, an interactive timeline T with blocks B that can be made larger, smaller or moved in time (horizontally and/or on the X-axis) is shown.

In this embodiment, the blocks B comprise the stimulation parameters.

In this embodiment, the interface module 116 is a graphical user interface.

In this embodiment, the interface module 116 is a touch screen.

In general, other embodiments of the graphical user interfaces are possible.

In this embodiment, graphical illustrations of a person, e.g. a patient walking are added, hinting at which leg is 'being programmed'.

### HOW BLOCKS B ARE CREATED ON A TIMELINE T

In general, the blocks B that are populated on the timeline T could represent muscles and/or muscle groups and/or stimulation blocks and/or even specific muscle fibers of a patient.

In this embodiment the stimulation blocks "left flexion" LFLEX, "left extension" LEXT, "right flexion" RFLEX and "right extension" REXT are shown.

The blocks B could be generally created by performing test-stimulation prior to starting the create exercises.

In such a process, physiological measurements or recordings (e.g. feedback information, e.g. recordings by at least one sensor and/or camera) could be combined with test-stimulation.

A neuromodulation system, especially a neurostimulation system for a patient could attempt to invoke a response from certain muscles by sending test-pulses, and a recording-system (e.g. a sensor of an external module 220) could measure the results back in.

When a specific muscle and/or muscle group and/or stimulation block and/or specific fiber is detected to respond in combination with other anatomical features with similar function (for muscles this is called agonists and synergists), or otherwise if the anatomical feature responds in isolation, then the system 10, 110, 210 can distill a block B for this with a certain clinically-relevant function.

In one example, a system 10, 110, 210 could be configured for providing test-stimulation on certain electrodes via a neuromodulation system, especially neurostimulation system for a patient and measure feedback information of certain muscles, e.g. "Iliopsoas" and "rectus femoris" on the left leg, e.g. responding strongly, via sensors, e.g. EMG sensors of an external module 220 (physiological measurements).

Alternatively, and/or additionally, an external module 220 could comprise at least one motion-capture camera, configured and arranged to detect these muscles to respond.

These muscles are both responsible for a locomotion (joint) action called "left hip flexion", which is essential to walking.

The system 10, 110, 210 could compose a block B from this stimulation, called "left hip flexion".

This block B could represent and/or comprise the stimulation parameters to activate the Iliopsoas and rectus femoris on the left leg.

Such a block B then could facilitate adding a time component by the timeline T.

Note that stimulation parameters for other muscles and/or muscle groups and/or stimulation blocks and/or certain muscle fibers could be obtained similarly.

In general, blocks B could additionally and/or alternatively contain information about antagonistic muscles.

For certain clinically relevant muscle stimulations, it is relevant that specific other muscles are not stimulated.

A block B could also contain information about which antagonistic muscle should not be excited by its stimulation parameters.

In general, muscles could have various types of relation to a certain movement or clinical function, which should be captured in the information of a block B:
A muscle could be an:
- an agonist
- an antagonist
- a synergist
- a neutralizer

In one embodiment, the system 10, 110, 210 could create a block B for flexing the lower arm (bending it).

For this, it uses the measurements in which it found settings to activate the biceps.

However, in its information the system 10, 110, 210 could also capture information about activation of the triceps, being a muscle that should certainly not be active to achieve this flexion of the arm.

### BLOCK B RAMPING

When blocks B are placed on the timeline T and an exercise is started for the patient, each block B could represent a stimulation with a certain set of stimulation parameters:
- amplitude (mA / mV)
- pulse frequency (Hz)
- pulse width (uS)

As soon as the block B onsets, the stimulation could be applied with these settings.

When the block B ends, the stimulation could drop immediately.

The clinical effect is that the patient immediately could experience the full strength of the stimulation when a block B starts.

The patient could be unprepared and overcome by this stimulation, frequently causing a violent muscular response (e.g. hip flexion).

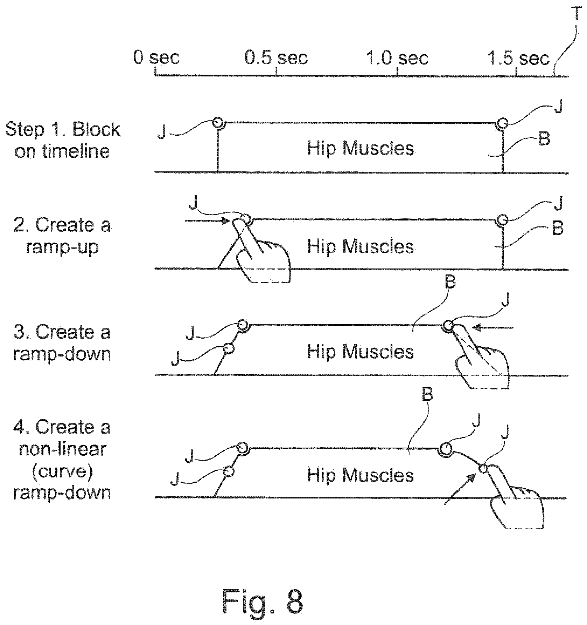

As a solution, a block B on the timeline could be modified to include ramping-up and/or ramping-down, cf. **Fig. 8****.**

### CYCLIC AND/OR REPEATING TIMELINET

A stimulation timeline T could be automatically repeated to allow for a constant motoric function, e.g. locomotion or autonomic function, to be performed.

Locomotion could be or could comprise e.g. walking, running, cycling, swimming.

The timeline T should behave like a 'automatically replay' in these exercises.

In general, the invention could be extended by describing three possible timeline T cycling and/or repeat behavior modes:
- Run once and then stop stimulation;
- Run once but keep stimulating the last stimulation block indefinitely until manually stopped by the user. This could be beneficial e.g. for standing up, where at the end stimulation should be facilitated to keep the patient standing upright;
- Run indefinitely until manually stopped a user.

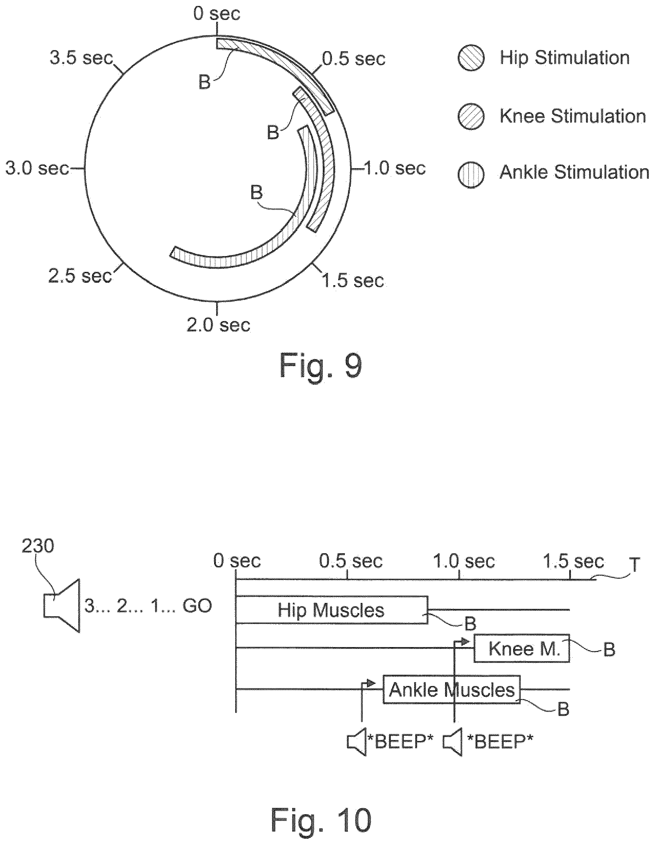

A timeline T could also be embodied to be circular, to make this cyclic behavior more visual, cf. **Fig. 9**, showing an example of a timeline T embodied as a circle that repeats itself.

### AUITORY CUES AND/OR TONES

To further assist a user to tune to timing (start and stop of stimulation of a muscle), the system 10, 110, 210 could play one or more auditory tones as acoustic information, e.g. as soon as a block B is initiated and/or stopped, cf. **Fig. 10**, showing an example of how acoustic information is provided, according to the present invention.

According to the invention the auditory tone is provided by the acoustic means 230.

In general, it is not required that the acoustic means 230 provides the auditory tone by itself.

For example, in a neuromodulation system, in particular in a neurostimulation system for a patient comprising a programmer, in particular a tablet used to program a pulse generator via an intermediate microprocessor, the auditory tone can be produced by any of these.

The auditory tone could be a simple tone and/or single frequency and/or multiple frequencies and/or music and/or a vocal cue such as for important muscles and/or muscle groups involved in the movement, e.g. "left rectus femoris".

In general, vocals could provide a count-down before stimulation is applied.

This could help the patient to align and prepare his self-invoked movements to align with these stimulations.

### MANUAL SEQUENCING OF BLOCKS B

As shown in earlier examples, the user could manually place blocks B on the timeline T and/or move these and/or increase and/or decrease their timing.

Alternatively, and/or additionally, templates could be provided for certain exercises and for patients with specific residual motor-functions.

In general, the user could manually change the stimulation parameters and/or the time sequence of stimulation parameters and/or the blocks on the timeline by acting on control elements J configured for being actuated by the user, including but not limited to axes, points, knots, buttons, arrows, hand signals, emojis, crosses and/or windows and/or text and/or shortcuts.

In general, control elements could also be referred to as joints J.

### AUTOMATED SEQUENCING OF STIMULATION PARAMETERS AND/OR BLOCKS B

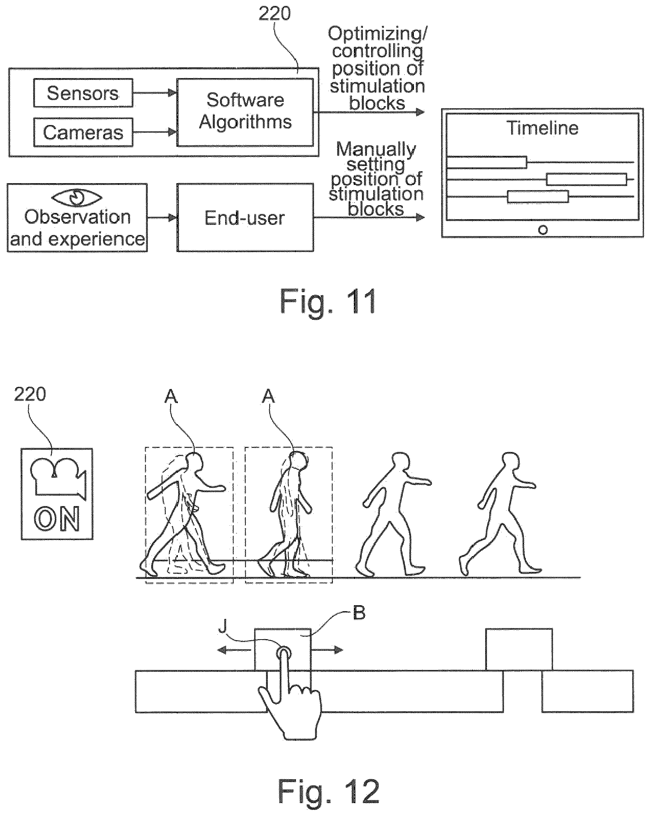

In addition to manual control, other elements, e.g. an external module 220 could control the contents of the timeline T, cf. **Fig. 11****.**

The external module 220 could fine-tune the stimulation parameters by using an algorithm.

Additionally, and/or alternatively, the external module 220 could fine-tune the stimulation parameters by providing feedback information.

In particular, the start and stop of stimulation parameters and/or up ramping and/or down ramping and/or duration and/or repetition and/or cycles and/or the position of a block B on the timeline could be controlled.

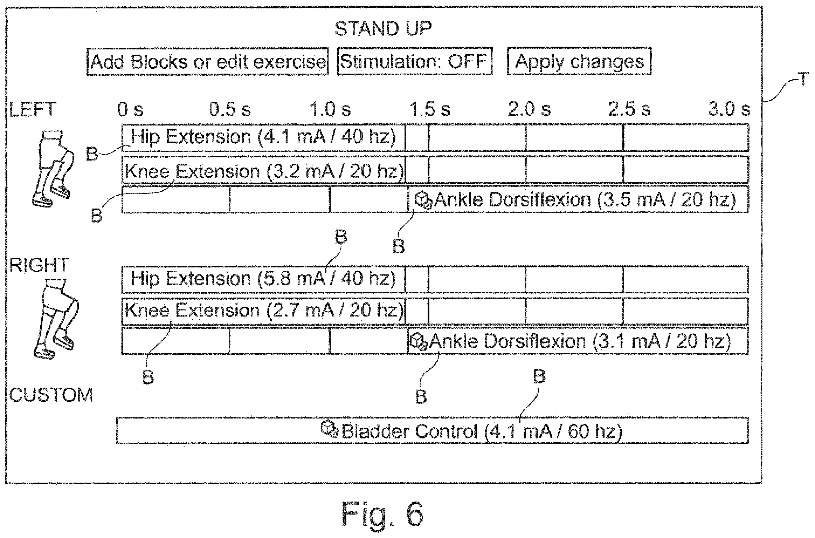

**Fig. 6** shows a further example of a graphical information provided by the graphical presentation module 14, 114, 214, according to the present invention.

Fig. 6 shows a timeline T concept for "stand up" locomotion exercise.

A timeline T concept is shown whereby left and right muscles are split up to make it more understandable.

The timeline T is split in muscular activation of the left side of the body, followed by activation of the right side of the body, to provide a more understandable and user-friendly overview.

Also, an autonomic function is included called bladder control.

In general, autonomic function can be alternatively and/or additionally be provided to the exercise.

In general, autonomic functions could be or could comprise at least one of bladder control, bowel control, sphincter control, sexual function, heart rate, blood pressure.

For certain exercises it could make more sense to place blocks B not with respect to time, but rather the progression of an exercise.

The progression of an exercise could be depicted for example as a percentage (0-100%, for e.g. closed-loop cycling, closed-loop walking), a sequence of limb positions (for e.g. closed-loop cycling, closed-loop sit-to-stand) or a sequence of exercise events (e.g. gait events).

For example, a knee extension could be associated to the arbitrary time interval between a bent knee (angle between thigh and shank at 90°) and an extended knee (thigh and shank in-line).

Some exercises could require a combination of the two methods (e.g. closed loop/triggered walking), where a time sequence is defined for certain exercise events (e.g. gait events).

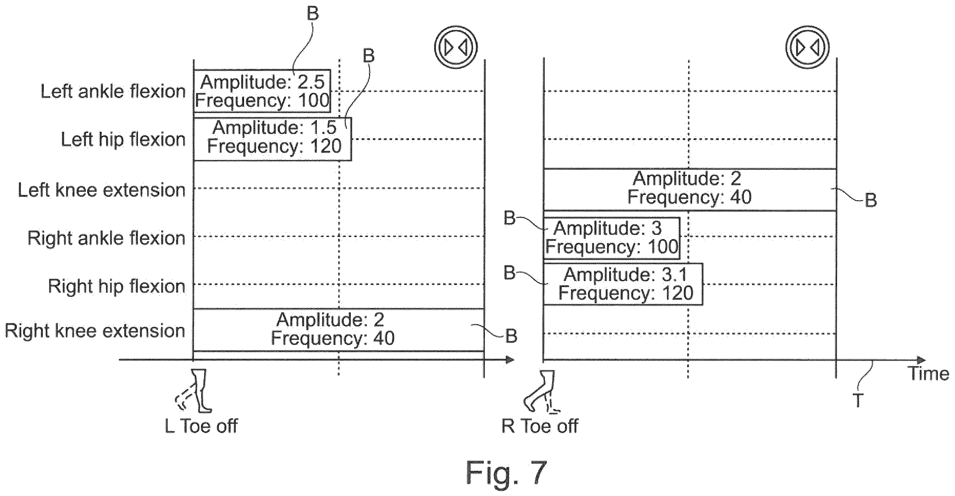

For example, a 400ms hip flexion is associated to a detected toe-off gait event, cf. **Fig. 7****,** showing a further example of graphical information provided by the graphical presentation module according to the present invention.

In general, a different visualization method could be a circular pattern that resembles a clock and more clearly illustrates that a timeline may be cyclic.

**Fig. 8** shows an example of how a user can interact on a touchscreen with a block B on the timeline T.

In this embodiment, the block B provides handles in the corner to directly manipulate the ramping duration.

In this embodiment the block B provides handles in the corner to directly manipulate the ramping angle (forming a non-linear curve).

This could be modified and set by the user, or by an automated process (e.g. set of instructions/algorithms), cf. **Fig. 11**, showing a schematical embodiment of how stimulation parameters could be updated according to the present invention.

Alternatively, this could be modified and set by the user and by an automated process (e.g. set of instructions/algorithms).

In this embodiment, any of the stimulation parameters can be gradually increased and/or decreased at the onset or offset of a block B.

In this embodiment, non-linear ramp up and/or ramp down is enabled.

This is much more natural to a patient and is foreseen to lead to better therapy with improved clinical outcome.

**Fig. 12** shows an example of superimposing camera recordings on intended locomotion of a patient, according to the present invention.

To provide the user a better guidance for improving the patient's stimulation parameters, e.g. camera recordings (provided by a camera comprised by the external module 220) and/or frames could be superimposed onto optimal outcome patterns.

Optimal outcome patterns could be movements of a healthy subject.

As such, the user could see which muscles need more and/or less activation to match e.g. an optimal walking pattern.

This could be applied to kind of locomotion activity and/or motoric function.

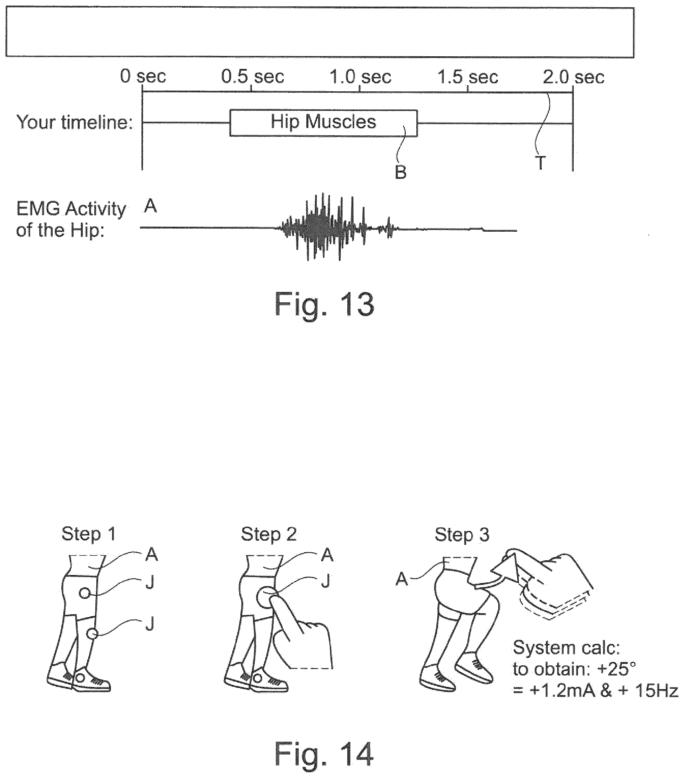

**Fig. 13** shows a further example of adding (and/or superimposing) physiological measurements to support block B positioning, according to the present invention.

Physiological measurements, in addition to or instead of camera-recordings, can also give information on how to position blocks B.

Sensors, e.g. EMG sensors may be applied on a patient to measure when muscles are activated voluntarily by the patient, and this could be displayed in the same sequence, e.g. timeline T as the one the blocks B are placed in.

In particular, a timeline T with hip muscle block B and live EMG measurements of that hip are shown.

The EMG trace data could be used to better position the block B to align with the patient's voluntary activity.

In general, sensors could be used to better position blocks B to align with the patient's voluntary activity.

As such, the user has guidance to position blocks B more effectively, and to the benefit of the therapy.

**Fig**. **14** shows an example of direct manipulation and/or modulation of a movement of a patient on an avatar A, according to the present invention.

The user may directly interact with an avatar A to modulate and configure the stimulation settings to obtain a certain movement.

An avatar A could be displayed with discrete joints J.

In general, an avatar A could be displayed with at least one joint J.

Joints J could be generally referred to as elements that can be controlled with stimulation.

The user could interact with the joints J and use touch-based interaction to increase or decrease a motion.

The system 10, 110, 210 could translate this motion to certain stimulation parameters.

### References

- 10,110,210: system
- 12, 112, 212: stimulation pattern programming module
- 14, 114, 214: graphical presentation module
- 116, 216: interface module
- 118,218: computer assisted module
- 220: external module
- 230: acoustic module

- A: avatar
- B: block
- FLEX: flexion
- EXT: extension
- J: joint
- L: left
- R: right
- T: timeline

## Claims

1. A planning and/or control system (10, 110, 210) for a neuromodulation system, especially a neurostimulation system for a patient, comprising
- a stimulation pattern programming module (12, 112, 212) configured and arranged for receiving and processing data on stimulation parameters and a time sequence of stimulation parameters,
- a graphical presentation module (14, 114, 214) configured and arranged for providing graphical information about the stimulation parameters and the time sequence of the stimulation parameters.

2. The system (10, 110, 210) according to claim 1, **characterized in that** the stimulation pattern programming module (12, 112, 212) is or comprises an interface module (116, 216) configured and arranged for receiving user-based stimulation parameters and/or a user-based time sequence of the stimulation parameters.

3. The system (10, 110, 210) according to claim 1 or claim 2, **characterized in that** the system (10, 110, 210) is configured such that the stimulation parameters and/or the time sequence of the stimulation parameters is modulated dependent on modulation of the graphical information about the stimulation parameters and the time sequence of the stimulation parameters.

4. The system (10, 110, 210) according to one of claims 1 to 3, **characterized in that** the stimulation pattern programming module (12, 112, 212) is or comprises a computer-assisted module (118, 218) configured and arranged for updating stimulation parameters and/or the time sequence of stimulation parameters based on feedback information provided by at least one external module (220) and/or a set of instructions.

5. The system (10, 110, 210) according to one of claims 1 to 4, **characterized in that** the graphical presentation module (14, 114, 214) is configured and arranged for providing graphical information about the time sequence of the stimulation parameters as a timeline T, a graph, a plot, a table, a circular pattern and/or a clock.

6. The system (10, 110, 210) according to one of claims 1 to 5, **characterized in that** the stimulation parameters comprise amplitude, frequency, and/or pulse width of the stimulation of at least one muscle and/or at least one stimulation block and the time sequence of stimulation parameters comprises start, stop, up ramping, down ramping, duration, repetition and/or cycles of stimulation of at least one muscle and/or stimulation block.

7. The system (10, 110, 210) according to one of claims 1 to 6, **characterized in that** the system (10, 110, 210) further comprises at least one of a display, a controller, a programmer, a communication module, a telemetry module, a stimulation device, an electrode, a sensor and/or a sensor network.

8. The system (10, 110, 210) according to one of the preceding claims, **characterized in that** the system (10, 110, 210) comprises an acoustic module (230) being configured and arranged for providing acoustic information about the stimulation parameters and/or the time sequence of the stimulation parameters.

9. A method for planning and/or controlling neuromodulation, especially neurostimulation for a patient, comprising at least the steps of:
- receiving and processing stimulation parameters and a time sequence of the stimulation parameters,
- providing graphical information about the stimulation parameters and the time sequence of the stimulation parameters.

10. The method according to claim 9, **characterized in that** the method further comprises the step of receiving user-based stimulation parameters and/or a user-based time sequence of the stimulation parameters.

11. The method according to claim 9 or claim 10, **characterized in that** the method further comprises the step of modulating the stimulation parameters and/or the time sequence of the stimulation parameters by modulation of the graphical information about the stimulation parameters and the time sequence of the stimulation parameters.

12. The method according to one of claims 9 to 11, **characterized in that** the method further comprises the step of updating stimulation parameters and/or the time sequence of stimulation parameters based on feedback information and/or a set of instructions.

13. The method according to one of claims 9 to 12, **characterized in that** the graphical information about the time sequence of the stimulation parameters is provided as a timeline T, a graph, a plot, a table, a circular pattern and/or a clock.

14. The method according to one of claims 9 to 13, **characterized in that** the stimulation parameters comprise amplitude, frequency, and/or pulse width of the stimulation of at least one muscle and/or at least one stimulation block and the time sequence of stimulation parameters comprises start, stop, up ramping, down ramping, duration, repetition and/or cycles of stimulation of at least one muscle and/or stimulation block.

15. The method according to one of claims 9 to 15, **characterized in that** the method further comprise the step of providing acoustic information about the stimulation parameters and/or the time sequence of the stimulation parameters.
